Europäisches Patentamt

European Patent Office

Office européen des brevets

(19)

(11) **EP 1 303 540 B1**

(12) **EUROPÄISCHE PATENTSCHRIFT**

(45) Veröffentlichungstag und Bekanntmachung des Hinweises auf die Patenterteilung:
**11.01.2006 Patentblatt 2006/02**

(21) Anmeldenummer: **01949496.2**

(22) Anmeldetag: **14.07.2001**

(51) Int Cl.:
*C07K 16/46* (2006.01)     *A61K 39/395* (2006.01)
*A61P 35/00* (2006.01)     *A61P 37/00* (2006.01)

(86) Internationale Anmeldenummer:
**PCT/EP2001/008147**

(87) Internationale Veröffentlichungsnummer:
**WO 2002/008291 (31.01.2002 Gazette 2002/05)**

(54) **MULTISPEZIFISCHES REAGENZ ZUR SELEKTIVEN STIMULIERUNG VON ZELLOBERFLÄCHENREZEPTOREN**

MULTI-SPECIFIC REAGENT FOR SELECTIVE STIMULATION OF CELL SURFACE RECEPTORS

REACTIF MULTISPECIFIQUE DESTINE A LA STIMULATION SELECTIVE DE RECEPTEURS DE SURFACE CELLULAIRE

(84) Benannte Vertragsstaaten:
**AT BE CH CY DE DK ES FI FR GB GR IE IT LI LU MC NL PT SE TR**

(30) Priorität: **20.07.2000 DE 10034607**

(43) Veröffentlichungstag der Anmeldung:
**23.04.2003 Patentblatt 2003/17**

(73) Patentinhaber: **Jung, Gundram**
**72108 Rottenburg-Wendelsheim (DE)**

(72) Erfinder: **Jung, Gundram**
**72108 Rottenburg-Wendelsheim (DE)**

(74) Vertreter: **Viering, Jentschura & Partner**
**Postfach 22 14 43**
**80504 München (DE)**

(56) Entgegenhaltungen:
EP-A- 0 336 379          WO-A-00/29431
WO-A-99/48527          US-A- 6 010 902

• F. EMMRICH ET AL.: "Selective stimulation of human T lymphocyte subsets by heteroconjugates of antibodies to the T cell receptor and to subset-specific differentiation antigens." EUROPEAN JOURNAL OF IMMUNOLOGY, Bd. 18, Nr. 4, April 1988 (1988-04), Seiten 645-648, XP001053418 Weinheim, Germany in der Anmeldung erwähnt

• A. ASHKENAZI ET AL.: "Safety and antitumor activity of recombinant soluble Apo2 ligand." THE JOURNAL OF CLINICAL INVESTIGATION, Bd. 104, Nr. 2, Juli 1999 (1999-07), Seiten 155-162, XP000973369 New York, NY, USA

• T. GRIFFITH ET AL.: "Functional analysis of TRAIL receptors using monoclonal antibodies." THE JOURNAL OF IMMUNOLOGY, Bd. 162, 1. März 1999 (1999-03-01), Seiten 2597-2605, XP000918935 Baltimore, MD, VSA in der Anmeldung erwähnt

• J. DHEIN ET AL.: "Induction of apoptosis by monoclonal antibody anti-Apo-1 class switch variants is dependent on cross-linking of Apo-1 cell surface antigens." THE JOURNAL OF IMMUNOLOGY, Bd. 149, Nr. 10, 15. November 1992 (1992-11-15), Seiten 3166-3173, XP002024490 Baltimore, MD, VSA in der Anmeldung erwähnt

• OGASAWARA J. ET AL: 'Lethal effect of the anti-Fas antibody in mice.' NATURE Bd. 364, 26 August 1993, LONDON, GB, Seiten 806 - 809

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung beschäftigt sich mit der selektiven Aktivierung von Rezeptoren auf der Oberfläche der Zelle. Insbesondere betrifft die Erfindung bispezifische, Fc-freie Antikörperfragmente mit einer monovalenten Bindungsstelle für einen Todesrezeptor und zumindest einer Bindungsstelle für ein tumorzellspezifisches Zelloberflächenantigen.

Es ist allgemein bekannt, daß extrazelluläre Signale über Rezeptorproteine durch die Plasmamembran übertragen werden, die dazu in der Lage sind, die extrazelluläre Bindung von Liganden in ein intrazelluläres biochemisches Ereignis umzusetzen. Auf diese Weise aktivieren Zelloberflächenrezeptoren intrazelluläre Signalpfade, die zu verschiedenen Stellen in der Zelle führen und dort ein bestimmtes Ereignis auslösen.

Alle Zelloberflächenrezeptoren sind transmembrane Proteine oder Proteinkomplexe, die eine Verbindung zwischen dem Inneren und dem Äußeren der Zelle herstellen. Viele Rezeptoren erfahren eine bestimmte Veränderung in der Proteinkonformation, wenn ihr jeweiliger Ligand gebunden wird. Bei einigen Rezeptortypen führt diese Konformationsänderung zur Öffnung eines Ionenkanales, während bei anderen Rezeptoren die Konformationsanderung dazu führt, daß der zytoplasmatische Bereich des Rezeptors so beeinflußt wird, daß er sich mit intrazellulären Signalproteinen und -enzymen assoziieren und diese aktivieren kann.

Ein entscheidender Effekt der Ligandenbindung an Rezeptoren besteht oft darin, den Rezeptor zu multimerisieren oder zu vernetzen und damit die eintrazelluläre Signalkaskade in Gang zu setzen. Eine solche Quervernetzung von Oberflächenrezeptoren kann entweder durch die physiologischen Liganden der Rezeptoren erfolgen oder z.B. in vitro durch entsprechende quervernetzende Antikörper.

Für Lymphozyten konnte zum Beispiel gezeigt werden, daß eine Stimulierung von spezifischen Antigenrezeptoren durch Bindung von F(ab')-Fragmenten, die nur eine Bindungsstelle haben, nicht möglich ist, während durch (Fab')$_2$-Antikörperfragmente, die also zwei Bindungsstellen aufweisen, die Rezeptoren Cluster bilden und die Zellen aktiviert werden. Eine stärkere Reaktion wird jedoch erreicht, wenn Lymphozyten durch intakte Antikörper stimuliert werden, die an Zellen gebunden sind, die Rezeptoren für den Fc-Teil von Immunglobulinen tragen. Mit anderen Worten, eine Aktivierung erfolgt, wenn Rezeptoren effektiv dadurch quervernetzt werden, daß sie an viele identische Antikörpermoleküle binden, die durch andere Zellen bereitgestellt werden, die Fc-Rezeptoren für die konstanten Regionen der intakten Antikörper aufweisen. Durch die so immobilisierten Antikörper werden die Rezeptoren effektiv quervernetzt.

In vitro läßt sich eine derartige Quervernetzung zum einen dadurch erreichen, daß die konstanten Bereiche von an die Rezeptoren gebundenen Antikörpern über Protein A oder über weitere Antikörper quervernetzt werden, die spezifisch an die konstanten Bereiche der an die Rezeptoren gebundenen Antikörper binden.

Für die Mitglieder der TNF (Tumor Nekrose Faktor)-Familie ist es z.B. bekannt, daß sie als Trimere wirken und daß die Liganden-induzierte Trimerisierung ihrer Rezeptoren das kritische Ereignis bei der Initialisierung der Signalübertragung ist.

Dhein et al., "INDUCTION OF APOPTOSIS BY MONOCLONAL ANTIBODY ANTI-APO-1 CLASS SWITCH VARIANTS IS DEPENDENT ON CROSS-LINKING OF APO-1 CELL SURFACE ANTIGENS", the Journal of Immunology, Band 149, 1992, Seiten 3166-3173, beschreiben, daß eine effektive Quervernetzung des APO-1 Zelloberflächenantigens zu einer Induktion der Apoptose führt. Sie zeigen, daß bei SKW6.4-Zellen Apoptose induziert wird, wenn an den APO-1-Rezeptor anti-APO-1 F(ab')$_2$-Fragmente binden, die über einen Schaf-anti-Maus-Antikörper quervernetzt werden, daß hierzu die Bindung der F(ab')$_2$ Fragmente allein aber nicht ausreicht. Aus diesen Ergebnissen schlußfolgern sie, daß die Bivalenz des Antikörpers, der also zwei Bindungsstellen für das APO-1-Zelloberflächenantigen aufweist, nicht ausreichend ist, um Apoptose zu induzieren, sondern daß hierzu eine effektive Quervernetzung des APO-1-Zelloberflächenantigens erforderlich ist.

Die Sequenz des APO-1-Antigens sowie ein monoklonaler Antikörper gegen das APO-1-Antigen sind beschrieben in US 5,891,434. Diese Patentschrift erwähnt, daß die Anti-APO-1-Antikörper verwendet werden können, um Tumore zu behandeln, die das APO-1-Antigen tragen, wobei die Antikörper ferner dazu verwendet werden können, in verschiedenen Typen von Zellen Apoptose zu induzieren.

Es ist jedoch bekannt, daß viele Zellen im Körper das APO-1-Zelloberflächenantigen tragen, so daß die Verabreichung eines Anti-APO-1-Antikörpers bei einem Tumorpatienten nicht nur zu einem Angriff auf die Tumorzellen, sondern darüber hinaus auch auf andere, gesunde und vielleicht sogar lebensnotwendige Zellen führt, die ebenfalls das APO-1-Oberflächenantigen tragen.

Ogasawara et al. konnten in einer grundlegenden Arbeit (*Nature* **364,** 806-809 (1993)) zeigen, dass ein monoklonaler *anti*-APO-1 Antikörper Wildtyp-Mäuse nach Injektion innerhalb von 2-6 h tötet. Die Mäuse wiesen dabei schwerste Leberschäden auf. Die Ursache dafür liegt in der Auslösung von Apoptose in den Hepatozyten durch die Bindung des *anti*-APO-1 Antikörpers an den APO-1 Rezeptor, der auf der Oberfläche von Leberzellen in hoher Dichte vorhanden ist, und der Induktion einer cytotoxischen Zellantwort, die wiederum über den Fc-Teil des Antikörpermoleküls vermittelt wird.

Vor diesem Hintergrund ist die Verwendung der bekannten *anti*-APO-1 Antikörper zur selektiven Abtötung von Tumorzellen, und damit zur Behandlung von Tumorpatienten, nicht oder nur bedingt geeignet.

Coney et al. (*Int. J. Cancer* **58,** 562-567 (1994)) beschreiben die Herstellung und Charakterisierung eines chimären (murin/human) *anti*-APO-1 Antikörpers einschließlich Fc-Teil, der in malignen Zellen Apoptose induzieren und dadurch die Regression von Tumoren vermitteln kann.

WO 94/20625 offenbart vollständige bispezifische Antikörper, die den Typ 1 TNF-Rezeptor (TNF-R1) und Fas-Antigen (synonym zu APO-1) binden und dadurch zur Lyse oder Wachstumshemmung maligner Zellen eingesetzt werden können. WO 99/48527 offenbart intakte bispezifische Antikörper, die gegen APO-2 und HER2 gerichtet sind und zu einer erhöhten Apoptose führen.

Ein weiterer Todesrezeptor, der durch Quervernetzung aktiviert wird, sind die TRAIL (TNF-related apoptosis-inducing ligand)-Rezeptoren R1 und R2; siehe Griffith et al., "Functional Analysis of TRAIL Receptors Using Monoclonal Antibodies", The Journal of Immunology, Band 162, 1999, Seiten 2597-2605. Die Autoren berichten, daß alle Anti-TRAIL-R2- und zwei der Anti-TRAIL-R1-Antikörper keine oder nur eine minimale Lysis von TRAIL-sensitiven Melanomzellen induzieren konnten, wenn sie den Zellen in Lösung zugegeben wurden, daß sie jedoch eine erhöhte lytische Fähigkeit zeigten, wenn sie an der Kulturplatte immobilisiert wurden, so daß sie für eine Quervernetzung der Todesrezeptoren TRAIL-R1- und -R2 sorgen konnten.

Auch Antiköper gegen die Todesrezeptoren TRAIL-R1 und -R2 wirken unspezifisch auf TRAIL-sensitive Zellen, so daß sie nur von geringem therapeutischem Nutzen sind.

Darüber hinaus ist es bekannt, daß sogenannte bispezifische Antikörper mit einer Spezifität für ein tumorassoziiertes Antigen und einer Spezifität für ein Oberflächen-Antigen von Abwehrzellen des Immunsystems, wie beispielsweise Makrophagen, T-Lymphozyten oder natürlichen Killerzellen, die über diese Bindung aktiviert werden, in der Immun-Krebstherapie eingesetzt werden können, um die Aktivität der Abwehrzellen auf die jeweiligen Targetzellen zu lenken.

Bispezifische Antikörper sind allgemein Antikörper, die zwei unterschiedliche Epitope binden können und für jedes Epitop monovalent sind. Hergestellt werden sie entweder mittels Oxidation von monovalenten F(ab')-Fragmenten zu einem F(ab')$_2$-Fragment, durch die Fusion von zwei Hybridomzellinien zu Hybrid-Hybridoma oder Quadroma-Zellen oder rekombinant.

Jung et al., "Target cell-induced T cell activation with biand trispecific antibody fragments", Eur. J. Immunol., Band 21, 1991, Seiten 2431-2435 beschreiben die Herstellung von bispezifischen F(ab)-Hybrid-Fragmenten, die für jedes Antigen monovalent sind.

Die Autoren zeigen, daß durch bispezifische Antikörper oder Fragmente eine durch die Zielzelle induzierte Aktivierung von T-Zellen erfolgen kann, indem die Antikörper einerseits an das Target-Antigen auf der Zielzelle und andererseits an den CD3-und/oder CD28-Rezeptor auf der T-Zelle binden.

Auch Segal et al., "Bispecific antibodies in cancer therapy", Current Opinion in Immunology, Band 11, 1999, Seiten 558-562 beschreiben ebenfalls die Verwendung von bispezifischen Antikörpern, durch die eine Effektorzelle auf eine Zielzelle gelenkt wird, die sie ansonsten nicht erkennen würde. Zu diesem Zweck binden die bispezifischen Antikörper an ein Oberflächenmolekül auf der Zielzelle sowie an einen Oberflächenrezeptor auf der Effektorzelle.

Roosnek et al., "T cell activation by a bispecific anti-CD3/anti-major histocompatibility complex class I antibody", Eur. J. Immunol., Band 20, 1990, Seiten 1393-1396 konnten zeigen, daß ein bispezifischer Antikörper mit einer Spezifität sowohl für MHC als auch für CD3 - beide auf T-Zellen exprimiert - eine effektive Proliferation von T-Zellen induzieren konnte, während eine Mischung der beiden Ursprungsantikörper hierzu nicht in der Lage war. Die Autoren hypothetisieren, daß dieser synergistische Effekt auf einer Störung der Verankerung des T-Zellrezeptor/CD3-Komplexes in der Membran beruht. Sie gehen dabei davon aus, daß der T-Zellrezeptor nicht unterscheiden kann, ob er an eine antigenpräsentierende Zelle (APC) oder an Oberflächenmoleküle innerhalb der eigenen Membran verankert wird. Sie spekulieren daher, daß der T-Zell-Rezeptor/CD3-Komplex, der in vivo durch Antigene auf einer anderen Zelle getriggert wird, auf Veränderungen in seiner Mobilität innerhalb der Membran reagiert.

Unklar geblieben ist jedoch der Mechanismus dieser T-Zellrezeptor-Aktivierung, die auf bestimmte T-Zell-Subpopulationen beschränkt ist. Der Stand der Technik ging bisher davon aus, daß es sich um einen T-Zell-Rezeptor-spezifischen Effekt handelt, der wahrscheinlich darauf beruht, daß neben dem T-Zellrezeptor ein Corezeptor wie CD4 oder CD8 stimuliert wird, der ebenfalls bei Stimulierung ein Signal erzeugt, siehe Emmrich et al., Eur. J. Immunol, 18,645 (1988).

Der vorliegenden Erfindung liegt demzufolge die Aufgabe zugrunde, neuartige Antikörperfragmente zur selektiven Abtötung von Tumorzellen ohne gleichzeitigen negativen Einfluss auf gesunde Zellen bereitzustellen. Gelöst wird dieses Problem durch die anspruchsgemäßen bispezifischen Fc-freien Antikörperfragmente mit einer monovalenten Bindungsstelle für einen Todesrezeptor und zumindest einer weiteren Bindungsstelle für ein tumorzellspezifisches Zelloberflächenantigen. Durch das Fehlen eines Fc-Teils wird verhindert, dass die Antikörperfragmente an Fc-Rezeptor tragende Zellen binden und so unselektiv Zelltod auslösen.

Zelloberflächenrezeptoren, die einer multimeren Stimulierung bedürfen, müssen nicht zwingend über immobilisierte oder z.B. durch Protein A oder an weitere Antikörper gebundene Antikörper quervernetzt werden. Bispezifische, Fc-freie Antikörperfragmente können, wie ein Rahmen der vorliegenden Erfindung erkannt, ebenfalls eine Targetantigen-restringierte Stimulierung des Zelloberflächenrezeptors induzieren.

Mit Hilfe der erfindungsgemäßen Antikörperfragmente ist es möglich, durch das Targetantigen einen bestimmten Zelltyp auszuwählen und auf diesem Zelltyp den entsprechenden Zelloberflächenrezeptor zu aktivieren wobei der Zelloberflächenrezeptor ein Todesrezeptor ist und das Targetantigen ein tumorzellspezifisches Zelloberflächenantigen ist. Von den beiden Bindungsstellen des Antikörperfragments ist damit die eine für die Funktion, nämlich den Zelloberflächenrezeptor, und die andere für die Spezifität, nämlich die Targetantigen-Restringierung verantwortlich. Nach Erkenntnis des Erfinders ergibt sich eine effektive Quervernetzung der Zelloberflächenrezeptoren auch und gerade dann, wenn beide Antigene auf derselben Zelle exprimiert werden. Dieses Ergebnis ist insofern überraschend, als aus dem Stand der Technik nicht klar war, auf welche weise bispezifische Antikörper für eine Quervernetzung zwischen einem Funktionsrezeptor und einem Targetrezeptor sorgen können, die zum Triggern des Funktionsrezeptors ausreicht, auch wenn beide Rezeptoren auf derselben Zelle exprimiert sind.

Der Erfinder der vorliegenden Anmeldung hat aus verschiedenen eigenen Experimenten abgeleitet, daß auf diese bispezifische Weise z.B. der Todesrezeptor APO-1 oder antigenpräsentierende Zellen (APCs) über die Stimulierung von CD40 (ein anderes Mitglied der TNF-Rezeptor Familie) unter Verwendung verschiedener Targetantigene, die auch eine Signalfunktion haben können, aber nicht müssen, stimuliert werden können wobei die Stimulierung von APCs kein Teil der Erfindung ist. Ausgehend von der Arbeit von Roosnek et al. a.a.O. sowie Emmrich et al a.a.O. war dies nicht zu erwarten, sondern bedurfte einer umfangreichen, experimentellen Verifizierung.

Durch Verwendung der erfindungsgemäßen Antilkörperfragmente ist es jetzt möglich, z.B. Todesrezeptoren auf bestimmten Targetzellen zu stimulieren, um auf diese Weise Krebszellen selektiv abzutöten oder aber im Rahmen einer Immunsuppression aktivierte T-Zellen, die Todesrezeptoren exprimieren, in den apoptotischen Zelltod zu treiben, wobei letztere Applikation kein Bestandteil der vorliegenden Erfindung ist.

Das erfindungsgemäße Antikörperfragment kann auch in einer pharmazeutischen Zusammensetzung mit einem pharmazeutisch akzeptierbaren Träger eingesetzt werden, denn die insbesondere bei Antikörpern gegen Todesrezeptoren schädliche Unspezifität wird erfindungsgemäß durch die Restringierung über das Targetantigen vermieden.

Nach welchen Verfahren pharmazeutisch akzeptierbare Träger, Formulierungen etc. ausgewählt werden können, ist beispielsweise in der eingangs erwähnten US 5,891,434 beschrieben.

Die wesentliche Anforderung, die das Antikörperfragment gemäß Anspruch 1 erfüllen muß, besteht darin, zwei Bindungsstellen bereitzustellen, nämlich eine für einen Todes rezeptor und eine weitere für ein tumorzellspezifisches Zelloberflächenantigen, die beide auf derselben Zelle exprimiert sind.

Die rekombinante DNA-Technik ermöglicht die Synthese unterschiedlicher bispezifischer Antikörper, nämlich von Tandemantikörpern und Diabodies. Bei Tandemantikörpern werden die Genfragmente für zwei scFv (single chain antigene binding proteins) über eine Linker-Sequenz verbunden und als ein Peptid synthetisiert. Bei Diabodies werden in einer Zelle zwei scFv exprimiert, die oft zu einer derartigen Dimerisation neigen, daß die variable Region der einen leichten Kette sich nicht an die variable Region "ihrer" schweren Kette, sondern an die des zweiten scFv-Moleküls anlagert, ohne daß sie kovalent verknüpft sind. Auf diese Weise lassen sich bispezifische Diabodies erzeugen, bei denen die DNA-Sequenzen der variablen Region der leichten Ketten der beiden zu kombinierenden scFv-Moleküle in den Expressionsvektoren gegeneinander ausgetauscht werden. Nach ihrer Synthese lagern sich die variablen Regionen der antigenspezifischen leichten und schweren Ketten aneinander, und es bildet sich ein rekombinantes Antikörper-Molekül mit zwei unterschiedlichen Spezifitäten.

Die Fusion von verschiedenen Bindungsdomänen an die scFv ermöglicht ein breites Spektrum von Kombinationsmöglichkeiten zweier scFv zu bispezifischen Antikörpern.

Es wird bevorzugt, wenn das Targetantigen (i.e. das tumorzellspezifische Zelloberflächenantigen) ebenfalls ein Zelloberflächenrezeptor ist, der zu seiner Stimulierung einer multimeren Ligandenbindung bedarf.

Bei dieser Maßnahme ist von Vorteil, daß das Targetantigen selbst ein aktivierbarer Zelloberflächenrezeptor ist, so daß die beiden Zelloberflächenrezeptoren (i.e. Todesrezeptor und tumorzellspezifisches Zelloberflächenantigen) gleichzeitig aktiviert und restringiert werden. Auf diese Weise kann auf der so ausgewählten Zelle z.B. ein synergistischer Effekt durch die gleichzeitige Stimulierung von zwei Zelloberflächenrezeptoren erreicht werden.

Der Todesrezeptor ist beispielsweise APO-1, TRAIL-R1 oder TRAIL-R2.

Diese Aufzählung der Todesrezeptoren ist lediglich beispielhaft, die Erfindung bezieht auch andere Todesrezeptoren mit ein, die über eine multimere Stimulierung ausgewählte Funktionen in den Targetzellen hervorrufen.

Das Targetantigen ist ein tumorzell-spezifisches Zelloberflächenantigen.

Hierzu bieten sich insbesondere CD-Marker an, durch die verschiedene Subpopulationen z.B. der Leukozyten bzw. deren verschiedener Entwicklungs- oder Differenzierungsstadien charakterisiert sind. Die ständig wachsende Liste der CD-Antigene umfaßt jedoch auch Moleküle, die auf anderen Zelltypen, z.B. Endothelzellen, Nervenzellen oder Fibroblasten zu finden sind; siehe hierzu Lexikon der Biologie, Spektrum Akademischer Verlag GmbH, Heidelberg, 1999, Band III, Seiten 323-329.

Das bispezifische Antikörperfragment der Erfindung ist auf diese Weise universell einsetzbar, indem über das Targetantigen eine Restringierung der durch die erste Bindungsstelle stimulierten Funktion des Todesrezeptors bewirkt wird.

Durch Verwendung der erfindungsgemäßen Antiköperfragmente kann in Zellen eine Targetantigenrestringierte Apoptose induziert werden. Somit können die Antikörperfragmente der Erfindung z.B. in der Immun-Krebstherapie eingesetzt werden.

Da das Targetantigen spezifisch auf Tumorzellen exprimiert wird, werden lediglich Tumorzellen selektiv abgetötet, während andere Zellen des Organismus, die ebenfalls den aktivierbaren Zelloberflächenrezeptor enthalten, nicht geschädigt werden, da es ihnen an dem Targetantigen fehlt.

Wenn das Targetantigen dagegen spezifisch auf T-Zellen exprimiert wird, wobei dies keinen Bestandteil der vorliegenden Erfindung darstellt, können die T-Zellen selektiv abgetötet werden, die Todesrezeptoren exprimieren. Dieses ist beispielsweise im Rahmen einer Immunsuppression bei einer Organtransplantation von Vorteil.

Wenn das Targetantigen spezifisch auf antigenpräsentierenden Zellen exprimiert wird, wobei dies keinen Bestandteil der vorliegenden Erfindung darstellt können diese APCs selektiv stimuliert werden, wenn das multispezifische Reagens neben der Bindungsstelle für das Targetantigen zumindest eine Bindungsstelle für CD40 aufweist.

Zusammenfassend läßt sich festhalten, daß es durch die Erfindung erstmals möglich geworden ist, die Aktivierung bestimmter Todesrezeptoren dadurch auf bestimmte Targetzellen zu beschränken, daß ein bispezifisches Fc-freies Antikörperfragment eingesetzt wird, das zumindest zwei Bindungsstellen aufweist, eine für einen zu stimulierenden Todesrezeptor und zumindest eine weitere für ein tumorzellspezifisches Zelloberflächenantigen auf derselben Zelle, über das die Targetzelle spezifiziert wird.

Weitere Vorteile ergeben sich aus der Beschreibung und der beigefügten Zeichnung:

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

Fig. 1      prinzipiell die Bindung von bispezifischen Antikörpern an zwei verschiedene Antigene auf derselben Zelle auf A) bizelluläre oder B) monozelluläre Weise;

Fig. 2      selektives Abtöten von SKW6.4-Zellen. nach er Inkubation von 16 Stunden mit einem erfindungsgemäßen bispezifischen Antikörperfragment mit einer Spezifität für CD20 und APO-1, während Jurkat-Zellen nicht abgetötet werden; und

Fig. 3      selektives Abtöten von SKW6.4- und Jurkat-Zellen durch erfindungsgemäße bispezifische Antikörper mit einer Spezifität für APO-1 und unterschiedlichen Targetantigenen.

Beispiel 1: Bindung bispezifischer Antikörper an Targetzellen

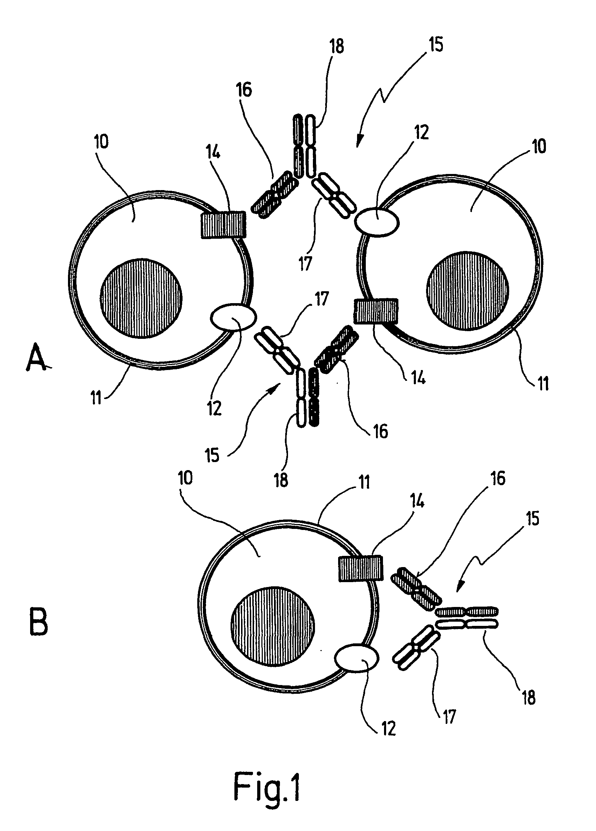

In Fig. 1 sind Targetzellen 10 dargestellt, auf deren Zelloberfläche 11 jeweils ein Todesrezeptor 12 sowie ein tumorzellspezifisches Zelloberflächenantigen 14 exprimiert sind.

An die Targetzellen 10 bindet ein intakter bispezifischer Antikörper 15 ,wobei der intakte Antikörper einschließlich Fc-Teil kein Bestandteil der vorliegenden Erfindung ist, dessen Fab-Fragment 16 eine Binduhgsstelle für das tumorzellspezifisches Zelloberflächenantigen 14 und dessen Fab-Fragment 17 eine Bindungsstelle für den Todesrezeptor 12 aufweist. Das Fc-Fragment ist an der Bindung sowie an einer Vernetzung nicht beteiligt weshalb in den unten beschriebenen Experimenten erfindungsgemäße Antikörperfragmente ohne FC-Teil, wie in Anspruch 1 definiert, verwendet werden konnten. Der bispezifische Antikörper 15, der die beiden Antigene 12 und 14 erkennt, bindet an beide Antigene auf derselben Zelle.

Eine Stimulierung der Todesrezeptoren 12 auf den Targetzellen 10 erfolgt nur dann, wenn das Fab-Fragment 16 gleichzeitig an ein tumorzellspezifisches Zelloberflächenantigen 14 binden kann.

Andererseits ist nicht auszuschließen, daß der bispezifische Antikörper 15 auf bizelluläre Weise zwei Targetzellen 10 miteinander verbindet, wie es in Fig. 1A gezeigt ist. Dies bedeutet, daß innerhalb einer Zellart, bei der jede Zelle 10 die beiden Antigene 12 und 14 aufweist, der bispezifische Antikörper 15 entweder unizellulär wie in Fig. 1B oder bizellulär wie in Fig. 1A binden kann, in jedem Fall aber zu einer Targetantigen-restringierten Stimulierung des Todesrezeptors 12 führt, der auf derselben Zelle vorhanden ist, wie das tumorzellspezifisches Zelloberflächenantigen 14.

Als Todesrezeptor 12 können verschiedene Todesrezeptoren, wie beispielsweise APO-1, TRAIL-R1 oder TRAIL-R2 dienen, wobei als tumorzellspezifisches Zelloberflächenantigen 14 beliebige tumorzellspezifische Zelloberflachenantigene verwendet werden können, durch die eine restringierte Stimulierung des Todesrezeptors 12 erreicht wird.

Mit anderen Worten, der Todesrezeptor 12 wird nur auf solchen Targetzellen 10 stimuliert, die entweder ein tumorzellspezifisches Zelloberflächenantigen 14 tragen oder sich bei bizellulärer Bindung in unmittelbarer Nähe einer solchen Targetzelle 10 befinden.

Dieses allgemeine Prinzip wird jetzt im folgenden anhand der Targetantigen-restringierten Stimulierung des Todesrezeptors APO-1 beschrieben.

Beispiel 2: Verwendete Zellinien

Als Zellinien werden SKW6.4- sowie Jurkat-Zellen verwendet. SKW6.4-Zellen (ATCC: TIB 215) stammen von B-Lymphozyten ab und exprimieren CD95 (APO-1), und sind Apoptose-sensitiv.

Jurkat-Zellen (ATCC: TIB 152) stammen von T-Lymphozyten ab, exprimieren ebenfalls CD95 und sind Apoptose-sensitiv.

Beide Zellinien werden in RPMI 1640 Medium, ergänzt durch 10 mM Glutamat, 100 U/ml Penicillin, 100 μg/ml Streptomycin und 10 % hitzeinaktiviertem fötalem Rinderserum (Sigma, Deisenhofen, Deutschland) inkubiert.

Als Zelloberflächenrezeptor wurde der auf beiden Zellinien exprimierte APO-1-Rezeptor (CD95) und als Targetantigen die CD-Marker CD2, CD5, CD19, CD20, CD28 und CD40 ausgewählt.

CD95-Antikörper können bei Santa Cruz Biotechnology, Santa Cruz, Kalifornien bezogen werden. Monoklonale Antikörper gegen die 6 verwendeten Targetantigene sind beispielsweise bei Biotrend Chemikalien GmbH, Eupener Straße 157, Köln kommerziell erhältlich.

Um die Expression von APO-1 und den 6 Targetantigenen zu überprüfen, wurden SKW6.4- und Jurkat-Zellen nach Inkubation mit, den entsprechenden Antikörpern (10 μg/ml) und FITC-markierten Antikörpern gegen Maus IgG (Dako, Hamburg, Deutschland) inkubiert. Die FACS-Analyse wurde mit einem FACS Calibur und der CelQuest-Software (Becton Dickinson, San Jose, Kalifornien) durchgeführt.

Es ergab sich, daß beide Zellinien APO-1 exprimieren, daß CD20 und CD40 und etwas schwächer CD19 auf SKW6.4 und CD28 sowie schwächer CD2 und CD5 auf Jurkat-Zellen exprimiert werden.

Beispiel 3: Herstellung bispezifischer Antikörperfragmente

Bispezifische Antikörperfragmente gemäß der Erfindung wurden durch selektive Reduktion und Re-Oxidation von Disulfidbrücken im Gelenkbereich hergestellt; siehe beispielsweise Jung et al. a.a.O. Die verwendeten Reaktionsbedingungen wurden so gewählt, daß die Bildung von Homodimeren verhindert wurde und eine nahezu vollständige Hybridisierung der modifizierten ursprünglichen Fab-Fragmente möglich war. Für die nachfolgenden Untersuchungen wurde die IgG2a-Variante des APO-1-Antikörpers mit Antikörpern hybridisiert, die gegen die Antigene CD19, CD20 und CD40 auf SKW6.4-Zellen sowie die Antigene CD2, CD5 und CD28 auf Jurkat-Zellen gerichtet sind.

In den Figuren werden die so hergestellten bispezifischen Antikörperfragmente durch ihre beiden Spezifitäten gekennzeichnet, die durch ein X voneinander getrennt sind.

Beispiel 4: Bestimmung der Targetantigen-restringierten Apoptose-Induktion

In den durchgeführten Versuchen sollte überprüft werden, ob die bispezifischen Antikörper-Fragmente gemäß der Erfindung den APO-1-Rezeptor nur auf solchen Targetzellen stimulieren konnten, die ebenfalls das jeweilige tumorzellspezifische Zelloberflächenantigen exprimierten, für welches das bispezifische Antikörper fragment ebenfalls eine Bindungsstelle aufwies.

Dieser Effekt wurde anhand der Abtötungsrate der Targetzellen bestimmt, wozu die Targetzellen (SKW6.4 und Jurkat) dreifach in 96$^{er}$ Platten (1 x 105/Kavität) mit 1 μg/ml des jeweiligen Antikörperkonstrukts inkubiert wurden.

Nach 16 Stunden Inkubation wurde die Lebensfähigkeit der verbliebenen Zellen unter Verwendung des Tetrazoliumsalzes WST-1 (Boehringer, Mannheim, Deutschland) bestimmt, das von mytochondrialen Enzymen umgesetzt wird und dabei ein dunkelrotes Formazan bildet.

Die optische Dichte wurde mit einem ELISA-Laser (SpektraMax 340, Molecular Devices, Sunnyville, Kalifornien) gemessen und der Prozentsatz an abgetöteten Zellen bei einer optische Dichte ODx wurde nach folgender Formel berechnet:

$$(1-ODx/ODmax) \times 100,$$

wobei ODmax die von Tumorzellen in Abwesenheit von Antikörpern erzeugte optische Dichte ist.

In einigen Experimenten wurde der Prozentsatz mit einem Chrom-Freigabe-Test bestimmt. Zu diesem Zweck wurden Zielzellen mit $^{51}$Cr markiertem Natriumchromat (80 μCi/ml, eine Stunde) inkubiert, intensiv gewaschen und dreifach in 96$^{er}$ Platten ausgesät. Nach einer Inkubation mit den Antikörpern für 16 Stunden wurde die angegebene Aktivität gezählt und der Prozentsatz an abgetöteten Zellen wie folgt berechnet:

$$cpm/cpm_{max} \times 100,$$

wobei $cpm_{max}$ die von mit einem Detergens behandelten Zielzellen freigegebene Radioaktivität ist.

Die mit den beiden unterschiedlichen Verfahren gemessenen Prozentsätze an abgetöteten Targetzellen entsprachen einander weitgehend.

Beispiel 5: Ergebnisse

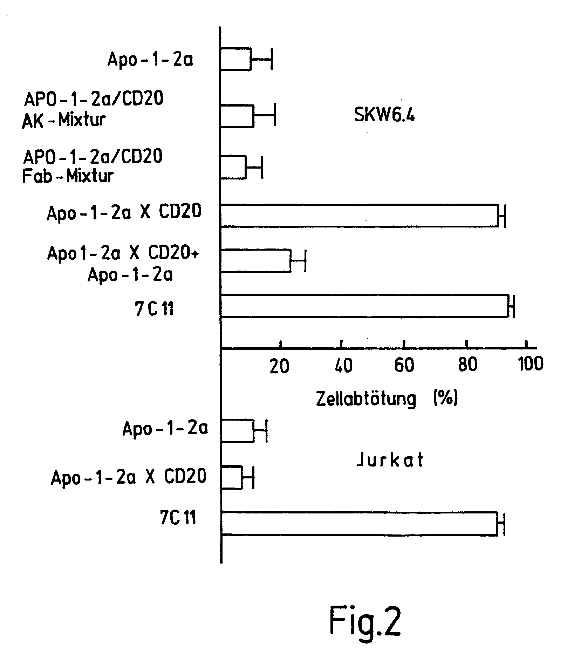

Fig. 2 zeigt, daß erfundungsgemäße bispezifische Antikörperfragmente mit einer Spezifität für APO-1 und CD20 (APO-1-2a x CD20) effektiv CD20-positive SKW6.4-Zellen, nicht aber die CD20-negativen Jurkatzellen abtöten konnten. Daß beide Zellinien sensitiv gegenüber einem APO-1-vermittelten Zelltod sind, ergibt sich aus der Tatsache, daß sie beide durch den Antikörper 7C11 abgetötet werden, der ein argonistischer IgM Antikörper (Immunotech, Marseille, Frankreich) ist, der Apoptose induziert.

Mixturen aus den beiden ursprünglichen Antikörpern, die entweder als intakte Antikörper oder als Fab-Fragmente eingesetzt wurden, konnten auch bei SKW6.4-Zellen keine Apoptose induzieren.

Darüber hinaus führte eine Koinkubierung des erfindungsgemäßen bispezifischen Antikörperfragmentes APO-1-2a x CD20 mit dem APO-1-2a-Antikörper zu einer Blockierung der durch das bispezifische Antikörperfragment vermittelte Lyse.

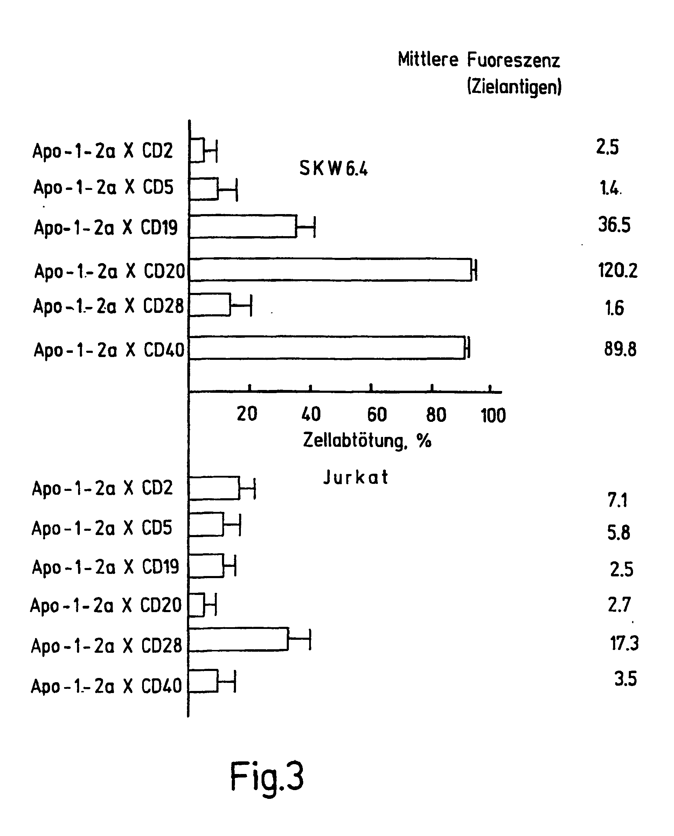

In Fig. 3, wo die FACS-Zahlen rechts Auskunft über die Expression des jeweiligen Targetantigens (i.e. des tumorzellspezifischen Zelloberfloberflächenantigens) auf den Zellen geben, ist zu erkennen, daß im wesentlichen die Menge an auf den Targetzellen exprimiertem Targetantigen für das Ausmaß der Abtötung der Targetzellen verantwortlich ist.

Eine signifikante Lyse von Jurkat-Zellen wurde nur von dem AP01-2a x CD28 Konstrukt erreicht.

APO-1-2a x CD2 bewirkte bei Jurkat-Zellen nur eine marginale Abtötung, wobei APO-1-2a x CD5 hier sogar völlig ineffektiv war. Sehr effizient waren dagegen APO-1-2a x CD20 und APO-1-2a x CD 40, die nahezu 100 %ige Abtötung von SKW6.4-Zellen bewirkten.

Weniger effektiv waren APO-1-2a x CD19 auf SKW6.4-Zellen und APO-1-2a x CD28 auf Jurkat-Zellen.

Vom Ergebnis her ist festzuhalten, daß Apoptose nur bei den Zellen induziert wurde, die außer dem APO-1-Rezeptor auch noch das entsprechende tumorzellspezifische Zelloberflächenantigen exprimierten.

**Patentansprüche**

1. Bispezifisches, Fc-freies Antikörperfragment mit einer monovalenten Bindungsstelle für einen Todesrezeptor und zumindest einer Bindungsstelle für ein tumorzellspezifisches Zelloberflächenantigen.

2. Antikörperfragment gemäß Anspruch 1, wobei der Todesrezeptor APO-1 ist.

3. Pharmazeutische Zusammensetzung, die ein bispezifisches, Fc-freies Antikörperfragment gemäß Anspruch 1 oder 2 enthält.

4. Verwendung eines bispezifischen, Fc-freien Antikörperfragments gemäß Anspruch 1 oder 2 zur Herstellung eines Medikamentes zur Abtötung von Tumorzellen.

**Revendications**

1. Fragment d'anticorps bispécifique, exempt de Fc, comportant un site de liaison monovalent pour un récepteur de mort et au moins un site de liaison pour un antigène de surface cellulaire spécifique de cellules tumorales.

2. Fragment d'anticorps selon la revendication 1, dans lequel le récepteur de mort est APO-1.

3. Composition pharmaceutique contenant un fragment d'anticorps bispécifique, exempt de Fc, selon la revendication 1 ou 2.

4. Utilisation d'un fragment d'anticorps bispécifique, exempt de Fc, selon la revendication 1 ou 2, pour la fabrication d'un médicament destiné à tuer des cellules tumorales.

**Claims**

1. Bispecific antibody fragment which is lacking a Fc moiety wherein the antibody fragment has a monovalent binding site for a death receptor and at least one binding site for a tumor cell-specific cell surface antigen.

2. Antibody fragment as defined in claim 1, wherein the death receptor is APO-1.

3. Pharmaceutical composition comprising a bispecific antibody fragment which is lacking a Fc moiety as defined in claim 1 or 2.

4. Use of a bispecific antibody fragment which is lacking a Fc moiety as defined in claim 1 or 2 for the manufacture of a medicament for the killing of tumor cells.

Fig.1

Fig.2

Mittlere Fuoreszenz
(Zielantigen)

SKW6.4

Apo-1-2a X CD2        2.5

Apo-1-2a X CD5        1.4.

Apo-1-2a X CD19        36.5

Apo-1-2a X CD20        120.2

Apo-1-2a X CD28        1.6

Apo-1-2a X CD40        89.8

20    40    60    80   100

Zellabtötung, %

Jurkat

Apo-1-2a X CD2        7.1

Apo-1-2a X CD5        5.8

Apo-1-2a X CD19        2.5

Apo-1-2a X CD20        2.7

Apo-1-2a X CD28        17.3

Apo-1-2a X CD40        3.5

Fig.3